# EUROPÄISCHE PATENTANMELDUNG

(11) **EP 4 725 604 A2**
(43) Veröffentlichungstag der Anmeldung: **15.04.2026**
(21) Anmeldenummer: 25207895.1
(22) Anmeldetag: 10.10.2025
(51) Int. Cl.: B01L 3/00

(54) **PROBENBEHÄLTER UND VERFAHREN ZUR HERSTELLUNG EINES PROBENBEHÄLTERS ZUR ZELLZÄHLUNG**

(30) Priorität: 14.10.2024 DE 102024129635
(71) Anmelder: Ritter GmbH, 86830 Schwabmünchen (DE)
(72) Erfinder: Schmuttermair, Stefan, 86853 Langerringen (DE)
(74) Vertreter: Lermer, Christoph

(57) **Zusammenfassung**

Eine Probenaufnahmevorrichtung 1 zur Aufnahme eines Fluids, insbesondere zum Zählen von Teilchen bzw. zur Bestimmung der Teilchenkonzentration in dem Fluid, insbesondere zur Bestimmung biologischer Zellen, weist zwei Probenaufnahmnekammern 11 und 12 mit einem definierten Aufnahmevolumen V auf. Die Probenaufnahmekammern 11 und 12 haben jeweils eine erste Öffnung 110, 120 zum Einbringen des Fluids in die jeweilige Kammer 11 bzw. 12 und eine Entlüftungsöffnung 111 bzw. 121. Die Probenaufnahmevorrichtung 1 besteht aus drei Komponenten A, B und C, wobei die dritte Kompnente C die erste Komponente A und die zweite Komponente B verbindet.

## Beschreibung

### TECHNISCHES GEBIET

Diese Anmeldung bezieht sich auf eine Probenaufnahmevorrichtung zur Aufnahme eines Fluids, insbesondere zum Zählen von Teilchen bzw. zur Bestimmung der Teilchenkonzentration in dem Fluid, insbesondere zur Bestimmung biologischer Zellen, umfassend: wenigstens eine Probenaufnahmnekammer mit einem definierten Aufnahmevolumen V; wobei die wenigstens eine Probenaufnahmekammer jeweils eine erste Öffnung zum Einbringen des Fluids in die jeweilige Kammer aufweist. Außerdem bezieht sich die Erfindung auf ein Verfahren zur Herstellung einer Probenaufnahmevorrichtung, insbesondere einer derartigen Probenaufnahmevorrichtung.

### STAND DER TECHNIK

Zur Bestimmung von Konzentrationen biologischer Zellen werden unterschiedliche Verfahren, beispielsweise basierend auf Fluoreszenz, eingesetzt. Bei der Zählung der Zellen bzw. der Bestimmung der Konzentration ist es erforderlich, ein genaues Fluidvolumen zu definieren und praktisch bereitzustellen. Zu diesem Zweck werden Probenaufnahmevorrichtungen aus Kunststoff mit eine Probenkammer, einer Zufuhröffnung und möglicherweise einem Entlüftungsauslass bereitgestellt. Die Aufnahmevorrichtung weist in der Regel eine plattenartige Struktur mit einer eingebetteten flachen Probenkammer auf. So können (ggf. beschichtete) Kunststofffolien z.B. durch Laserschweißen miteinander verbunden werden und im Zwischenbereich zwischen den Folien eine Probenkammer definieren.

Eine Herausforderung in der Kunststoffherstellung ist jedoch die hohe Genauigkeit, die bzgl. des Volumens der Probenkammer erforderlich ist.

### AUFGABE DER ERFINDUNG

Ausgehend davon besteht eine Aufgabe der vorliegenden Erfindung darin, eine Probenaufnahmevorrichtung mit Probenkammern mit definiertem Volumen und ein Verfahren zur Herstellung derselben bereitzustellen.

### BESCHREIBUNG DER ERFINDUNG

Diese Aufgabe wird gelöst durch eine Probenaufnahmevorrichtung zur Aufnahme eines Fluids, insbesondere zum Zählen von Teilchen bzw. zur Bestimmung der Teilchenkonzentration in dem Fluid, insbesondere zur Bestimmung der Konzentration biologischer Zellen, nach Anspruch 1, und durch ein Verfahren zur Herstellung der Probenaufnahmevorrichtung nach Anspruch 11 oder 12. Vorteilhafte Ausführungsformen ergeben sich aus den Merkmalen der abhängigen Ansprüche.

Eine erfindungsgemäße Probenaufnahmevorrichtung zur Aufnahme eines Fluids, insbesondere zum Zählen von Teilchen bzw. zur Bestimmung der Teilchenkonzentration in dem Fluid, insbesondere zur Bestimmung biologischer Zellen, umfasst: wenigstens eine Probenaufnahmnekammer mit einem definierten Aufnahmevolumen V; wobei die Probenaufnahmevorrichtung wenigstes zwei als Kunststoffteile hergestellte, zusammengefügte Komponenten (erste Komponente A, zweite Komponente B) umfasst, wobei die zwei Komponenten (A, B) die Probenaufnahmekammer (11, 12) begrenzen.

Die Probenaufnahmekammer(n) kann/können jeweils eine erste Öffnung zum Einbringen des Fluids in die jeweilige Kammer und/oder eine Entlüftungsöffnung aufweisen.

Insbesondere ist eine dritte Komponente (C) als Verbindungskomponente an die beiden anderen Komponenten angespritzt derart, dass sie mit der ersten und der zweiten Komponente in Kontakt tritt und eine kraft-, stoff- und/oder formschlüssige Verbindung zwischen der ersten und der zweiten Komponente herstellt.

Die Komponenten sind insbesondere Spritzgussbauteile. Die Komponenten A und/oder B können jedoch auch als gestanzte und/oder geprägte Kunststofffolien in ein Spritzgusswerkzeug eingelegt und dann überspritzt werden.

Insbesondere ist die erste Komponente (A) ein Kunststoffteil, bevorzugt ein Spritzgussteil, mit einem Grundkörper und wenigstens einer in der Oberseite des Grundkörpers ausgebildeten wannenartige Aussparung, deren Wände die Probenaufnahmekammer begrenzen.

Die Aussparung weist insbesondere eine vorgegebene, mit hoher Genauigkeit gefertigte Tiefe T auf.

Die zweite Komponente (B) kann eines oder mehrere Kunststoffteile, bevorzugt Spritzgussteile, aufweisen, welche die Aussparungen der Komponente A abdecken.

Die zweite Komponente (B) kann eine oder mehrere Abdeckungen bilden, die die eine bzw. die mehreren wannenartigen Aussparungen abdecken.

Insbesondere begrenzen die Wände der Aussparungen und die Abdeckung(en) die Probenaufnahmekammer(n).

Die Befestigung der zweiten Kompnente (B) an der ersten Komponente (A) erfolgt durch eine weitere ein- bzw. angespritzte dritte Komponponete aus Kunststoff, bevorzugt eine Spritzgusskomponente (C).

Die dritte Komponponete, wählweise eine Spritzgusskomponente (C), kann insbesondere durch Einspritzen in Zwischenräume zwischen den zweiten Komponenten (B) bzw. in einem Bereich um den Rand der zweiten Komponente(n) (B) herum auf die Oberfläche der Komponente (A) erfolgen.

Die dritte Komponponete, wählweise eine Spritzgusskomponente (C), kann auch durch An- bzw. Einspritzen in eine oder mehrere in der zweiten Komponente (B) ausgebildeten Nuten erfolgen, wobei die Nut(en) zur Oberfläche der ersten Komponente (A), die die wannenartige Aussparung aufweist, hin ausgerichtet ist.

In einem erfindungsgemäßen Verfahren zur Herstellung einer Probenaufnahmevorrichtung, insbesondere wie oben beschrieben, werden folgende Schritte durchgeführt: (a) Herstellen der Komponente (A) in einem Fertigungsverfahren, bevorzugt in einem Spritzgussverfahren, (b) Herstellen der zweiten Komponente(n) (B) in einem Fertigungsverfahren, bevorzugt in einem Spritzgussverfahren, (c) Auflegen der Komponente(n) (B) zum Abdecken der Öffnung(en) der Aussparung(en) der ersten Komponente (A), und (d) Einbringen der dritten Komponente (C) in Zwischenräume zwischen den zweiten Komponenten (B) bzw. in einen Bereich um den Rand der wenigstens einen zweiten Komponente(n) (B) herum auf die Oberfläche der ersten Komponente (A).

In einem erfindungsgemäßen Verfahren zur Herstellung einer Probenaufnahmevorrichtung, insbesondere wie oben beschrieben, werden folgende Schritte durchgeführt: (a) Herstellen der ersten Komponente (A) in einem Extrusions- oder Gieß- bzw. Gussverfahren, bevorzugt in einem Spritzgussverfahren, (b) Herstellen der zweiten Komponente(n) (B) in einem Extrusions- oder Gieß- bzw. Gussverfahren, bevorzugt in einem Spritzgussverfahren, (c) Auflegen der zweiten Komponente(n) B zum Abdecken der Öffnung(en) der Aussparungen der ersten Komponente (A), wobei wenigstes eine insbesondere in der zweiten Komponente ausgebildete Nut zur Oberfläche des Grundkörpers ausgerichtet ist, und (d) Einbringen der dritten Komponente (C) in die Nut der zweiten Komponente (B) auf die Oberfläche der ersten Komponente (A), wobei das Einbringen vorzugsweise über wenigstens eine Öffnung erfolgt, die die Nut mit der exponierten Oberseite der zweiten Komponente (B) verbindet.

Insbesondere werden die erste Komponente A und die zweite(n) Komponente(n) B in den Verfahrensschritten (a) und (b) zeitgleich in einem (demselben) Werkzeug hergestellt.

Die im Wesentlichen aus den drei Komponenten (A, B, C) bestehende Probenaufnahmevorrichtung wird insbesondere in einem Fügeverfahlen in Transfertechnik, mindestens jedoch einstufig, bevorzugt in einem Mehrkompopnentenspritzgussverfahren, hergestellt.

Die Herstellung der im Wesentlichen aus den drei Komponenten (A, B, C) bestehenden Probenaufnahmevorrichtung kann nacheinander oder in einem mehrstufigen Spritzgussverfahren erfolgen.

Die Komponenten (A, B, C), insbesondere die erste und zweite Komponente (A, B), können aus demselben Material gefertigt sein, und/oder das Material der dritten Komponente (C) kann gleich oder unterschiedlich von den Materialien der ersten Komponente A und/oder der zweiten Komponente B sein.

### KURZE BESCHREIBUNG DER FIGUREN

Weitere Merkmale und Vorteile der Erfindung werden anhand der nachfolgenden Beschreibung mit Bezug auf die Figuren deutlich. Es zeigen:
Fig. 1 zeigt eine perspektivische Ansicht einer erfindungsgemäßen Probenaufnahmevorrichtung;
Fig. 2 zeigt eine Draufsicht und eine Schnittansicht A-A einer ersten Ausführungsform der Erfindung;
Fig. 2A zeigt einen vergrößerten Ausschnitt der Schnittansicht aus Fig. 1;
Fig. 3 zeigt eine Draufsicht und eine Schnittansicht A-A einer zweiten Ausführungsform der Erfindung;
Fig. 3A zeigt einen vergrößerten Ausschnitt der Schnittansicht aus Fig. 3;
Fig. 3B zeigt einen vergrößerten Ausschnitt der Draufsicht aus Fig. 3;
Fig. 4 zeigt schematisch ein erfindungsgemäßes Herstellungsverfahren.

### BESCHREIBUNG BEVORZUGTER AUSFÜHRUNGSBEISPIELE DER ERFINDUNG

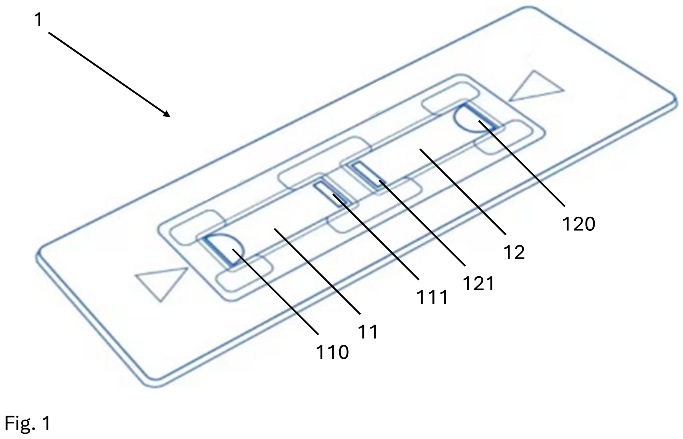

Die Figur 1 zeigt eine Probenaufnahmevorrichtung 1 zur Aufnahme eines Fluids, insbesondere zum Zählen von Teilchen bzw. zur Bestimmung der Teilchenkonzentration in dem Fluid, insbesondere zur Bestimmung biologischer Zellen. Die Probenaufnahmevorrichtung 1 kann als Objektträger für die Zellzählung eingesetzt werden.

Die Vorrichtung 1 weist zwei Probenaufnahmnekammern 11 und 12 mit einem definierten Aufnahmevolumen V auf. Die Probenaufnahmekammern 11 und 12 haben jeweils eine erste Öffnung 110, 120 zum Einbringen des Fluids in die jeweilige Kammer 11 bzw. 12 und eine Entlüftungsöffnung 111 bzw. 121.

Die Anzahl der Kammern ist nicht auf zwei beschränkt, sondern es können in einer Vorrichtung auch nur eine mehrere Kammern vorhanden sein.

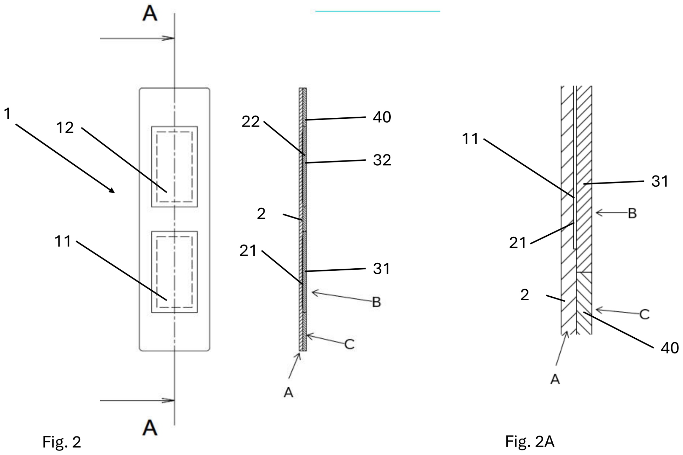

Die Figuren 2, 2A zeigen eine erste Ausführungsform einer Probenaufnahmevorrichtung 1 wie oben beschrieben. Der Einfachheit halber sind die Öffnungen nicht dargestellt.

Die Probenaufnahmevorrichtung 1 ist aus drei (3) Kunststoffkomponenten A, B und C hergestellt. Die Kunststoffe A und B sind in der Regel rigide.

Die Komponente A ist ein relativ flaches Spritzgussteil mit einem Grundkörper 2 und zwei in der Oberseite des Grundkörpers 2 ausgebildeten wannenartigen Aussparungen 21, 22, die die Probenaufnahmekammern 11 bzw. 12 unten und seitlich begrenzen. Die Aussparungen weisen eine vorgegebene, mit hoher Genauigkeit gefertigte Tiefe T, beispielsweise 95 µm, auf.

Die zweite Komponente B ist bzw. besteht aus einem oder mehreren ebenfalls flachen Spritzgussteilen 31, 32, die "Fenster" bilden, welche die Aussparungen 21, 22 der ersten Komponente A abdecken. Im Fall der Figur 2 sind zwei Spritzgussteile bzw. Abdeckungen 31, 32 vorhanden, die die wannenartigen Aussparungen 21 bzw. 22 abdecken. Die Wände der Aussparungen 21, 22 und die Abdeckungen 31, 32 begrenzen die Probenaufnahmekammern 11 und 12. Die Unterseiten der Abdeckungen 31, 32 sind (flächenmäßig) kleiner als die Öberfläche des Grundkörpers 2, jedoch größer als die Öffnungen der Aussparungen 21, 22, sodass sie über den Rand der Öffnungen der Aussparungen 21, 22 hinausragen und dort aufliegen. In den Abdeckungen 31, 32 ist in der Regel jeweils wenigstens eine erste Öffnung zum Einbringen des Fluids (nicht dargestellt) ausgebildet. Der Kunststoff der Komponente B ist (wenigstens bis zu einem bestimmten Grad) transparent, um die Ausführung optischer Zählverfahren zu ermöglichen. Insbesondere im Kunststoff der Komponente A können Additive eingearbeitet sein, die im Messverfahren benötigt werden. Dadurch kann sich ein möglicherweise notwendiger Schritt einer Beschichtung des Kunststoffs erübrigen.

Die Detaildarstellung Figur 2A zeigt insbesondere eine Aussparung 21 und die zugehörige Abdeckung 31.

Die Befestigung der zweiten Kompnente B an der ersten Komponente A erfolgt durch eine weitere angespritzte dritte Spritzgusskomponente C. In der ersten Ausführungsform sind die Abedeckungen 31, 32 der Wannen 21, 22 rechteckig und beabstandet voneinenader auf der Oberfläche des Grundkörpers 2 angeordnet. Zwischen die Abdeckungen wird nun durch Anspritzen (z.B. in einem 2- (2K) oder Mehr-Komopnenten Spritzgussverfahren) auf die Oberfläche des Grundkörpers 2 die weitere dritte Kompnente C eingebracht, die die Komponenten A und B (und C) verbindet. Die dritte Komponente C bildet eine Verbindungskomponente 40. Die Verbindung kann stoffschlüssig sein (Verbundspritzguss bei thermodynamisch verträglichen Kunststoffen A-C und B-C) oder formschlüssig (Montagespritzguss bei nicht verträglichen Kunststoffen A-C und/oder B-C).

In einem ersten Verfahren zur Herstellung einer Probenaufnahmevorrichtung werden folgende Schritte durchgeführt: (a) Herstellen der ersten Komponente A in einem Spritzgussverfahren, (b) Herstellen der zweiten Komponente(n) B in einem Spritzgussverfahren, (c) Auflegen der zweiten Komponente(n) B zum Abdecken der Öffnungen der Aussparungen 21 bzw. 22 der ersten Komponente A, und (d) Einbringen der dritten Komponente C in die Zwischenräume zwischen den Komponenten B bzw. in einem Bereich um den Rand der wenigstens einen Komponente B herum auf die Oberfläche der ersten Komponente A. Dadurch werden die Komponenten A, B und C verbunden.

Die exponierte Oberfläche der dritten Komponente C ist insbesondere bündig mit der exponierten Oberfläche der zweiten Komponente B ausgebildet.

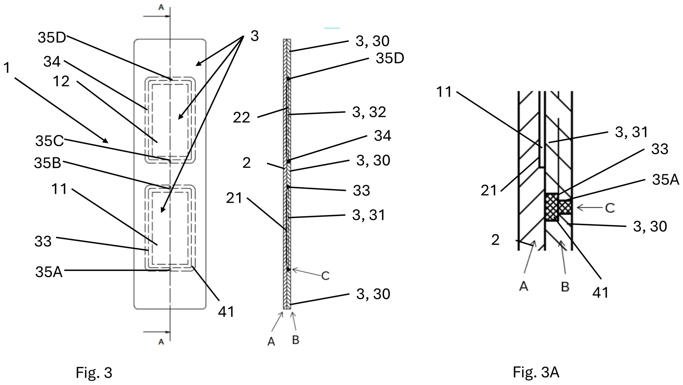

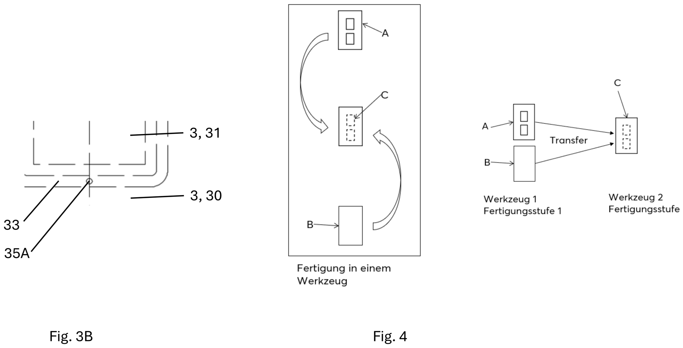

Die Probenaufnahmevorrichtung 1 kann insgesamt aus drei (3) Komponenten in einem Mehrkompopnentenspritzgussverfahren hergestellt werden. Durch den Aufbau und das Herstellungsverfahren wird eine präzise Herstellung der Probenvolumina V gewährleistet. Die Figuren 3 und 3A zeigen eine zweite Ausführungsform der Erfindung, wobei dieselben Merkmale/Komponenten mit denselben Bezugszeichen gekennzeichnet sind wie im ersten Ausführungsbeispiel. Im Unterschied zur ersten Ausführungsform ist die zweite Komponente B nicht in Form einzelner plattenartiger Abdeckungen ausgebildet, die auf der ersten Komponente A nebeneinander liegen, sondern als durchgängiges Element bzw. durchgängige Platte 3 in etway derselben Größe wie die Oberfläche des Grundkörpers 2. Die Platte 3 ist jedoch durch zwei zur Seite der Oberfläche des Grundkörpers 2 gerichtete Nuten 33, 34 aufgeteilt in zwei Abdeckungsbereiche 31, 32, ähnlich denend er ersten Ausführungsform, und einem Randplattenbereich 30, der die Abdeckungsbereiche 31, 32 umibt. Die Nuten 33, 34 erstrecken sich jeweils um die Abdeckungsbereiche 31, 32 herum. An bestimmten Stellen der Nuten sind Durchgangsöffnungen 35A-35D angeordnet, die die Nuten mit der exponierten Oberfläche der Komponente B verbinden.

Dies ist in der Figur 3B vergrößert dargestellt. Figur 3B zeigt die nach oben exponierte Öffnung 35A und die darunter liegende Nut 33 in der Platte 3. In der Platte 3 sind Nuten 33, 34 eingebracht, welche nach Außen mit kleinen Bohrungen 35A-35C verbunden sind, durch die die dritte Komponente C in Form einer Verbindungskomponente 41 ein- bzw. angespritzt wird. Die Verbindung ist entsprechend der im Zusamenhang mit der ersten Ausführungsform geschilderten Verbindung konzipiert. Das Verfahren für die Herstellung der zweiten Ausführungsform ist ebenfalls im Wesentlichen dasselbe wie das Verfahren für die Herstellung der ersten Ausführungsform.

In einem zweiten Verfahren zur Herstellung einer Probenaufnahmevorrichtung werden folgende Schritte durchgeführt: (a) Herstellen der ersten Komponente A in einem Spritzgussverfahren, (b) Herstellen der zweiten Komponente(n) B in einem Spritzgussverfahren, (c) Auflegen der zweiten Komponente(n) B zum Abdecken der Öffnung(en) der Aussparungen 21 bzw. 22 der ersten Komponente A, wobei Nuten 33, 34, die insbesondere in der zweiten Komponente B ausgebildet sind, zur Oberfläche des Grundkörpers 2 gerichtet sind, und (d) Einbringen der dritten Komponente C in die Nuten 33, 34 der zweiten Komponente B auf die Oberfläche der ersten Komponente A, wobei das Einbringen über Öffnungen 35A-35D erfolgt, die die Nut(en) mit der exponierten Oberseite der zweiten Komponente B verbinden. Dadurch werden die Komponenten A, B und C verbunden.

Das erste Verfahren ist schematisch in der Figur 4 dargestellt. Die erste Komponente A und die zweite Komponente(n) B werden in einem ersten Fertigungsschritt im Spritzguss in einem ersten Werkzeug hergestellt. Nach dem Abkühlen werden die Komponenten A und B in einem zweiten Fertigungsschritt übereinander gelegt und durch An- bzw. Einspritzen der dritten Komponente C in einem zweiten Werkzeug miteinander verbunden. Der Fertigungsprozess kann nacheinander, oder in einem mehrstufigen Spritzgussverfahren erfolgen.

Idealerweise werden die Komponenten A und B aus demselben Material gefertigt. Das Material der dritten Komponente C kann gleich oder unterschiedlich von dem Material der ersten Komponente (A) und/oder dem Material der zweiten Komponente (B) sein

## Patentansprüche

1. Probenaufnahmevorrichtung (1) zur Aufnahme eines Fluids, insbesondere zum Zählen von Teilchen bzw. zur Bestimmung der Teilchenkonzentration in dem Fluid, insbesondere zur Bestimmung biologischer Zellen, umfassend:
wenigstens eine Probenaufnahmnekammer (11, 12) mit einem definierten Aufnahmevolumen V;
wobei die Probenaufnahmevorrichtung (1) wenigstes zwei als Kunststoffbauteil, insbesondere Spritzgussbauteil, hergestellte, zusammengefügte Komponenten, nämlich eine erste Komponente (A) und wenigstens eine zweite Komponente (B) umfasst, wobei die erste Komponente (A) und die wenigstns eine zweite Komponente (B) die wenigstens eine Probenaufnahmekammer (11, 12) begrenzen.

2. Probenaufnahmevorrichtung (1) nach Ansporuch 1, wobei eine dritte Komponente (C) als Verbindungskomponente (40, 41) angespritzt wird derart, dass sie mit der ersten Komponente (A) und der zweiten Komponente (B) in Kontakt tritt und eine stoff- und/oder formschlüssige Verbindung zwischen der ersten und der zweiten Komponente herstellt.

3. Probenaufnahmevorrichtung (1) nach Ansporuch 1 oder 2, wobei die erste Komponente (A) ein Kunststoffbauteil, insbesondere ein Spritzgussteil, mit einem Grundkörper (2) und wenigstens einer in der Oberseite des Grundkörpers (2) ausgebildeten wannenartigen Aussparung (21, 22) ist, deren Wände die wenigstens eine Probenaufnahmekammer (11, 12) begrenzen.

4. Probenaufnahmevorrichtung (1) nach einem der vorhergehenden Ansprüche, wobei die Aussparung (21, 22) eine vorgegebene, mit hoher Genauigkeit gefertigte Tiefe T aufweist.

5. Probenaufnahmevorrichtung (1) nach einem der vorhergehenden Ansprüche, wobei die zweite Komponente (B) eines oder mehrere Kunststoffbauteile, insbesondere Spritzgussteile (3, 31, 32), aufweist, welche die Aussparung bzw. die Aussparungen (21, 22) der ersten Komponente A abdecken.

6. Probenaufnahmevorrichtung (1) nach einem der vorhergehenden Ansprüche, wobei die zweite Komponente (B) bzw. die zweiten Komponenten (B) eine bzw. mehrere Abdeckungen (31, 32) bilden, die die eine bzw. die mehreren wannenartigen Aussparungen (21, 22) abdecken.

7. Probenaufnahmevorrichtung (1) nach einem der vorhergehenden Ansprüche, wobei die Wände der Aussparungen (21, 22) und die eine bzw. die mehreren Abdeckungen (31, 32) die Probenaufnahmekammer (11, 12) bzw. die Probenaufnahmekammern (11, 12) begrenzen.

8. Probenaufnahmevorrichtung (1) nach einem der vorhergehenden Ansprüche, wobei die Befestigung der zweiten Kompnente(n) (B) an der ersten Komponente (A) durch eine weitere angespritzte dritte Spritzgusskomponente (C) erfolgt.

9. Probenaufnahmevorrichtung (1) nach einem der vorhergehenden Ansprüche, wobei die dritte Spritzgusskomponente (C) durch Einspritzen in Zwischenräume zwischen den zweiten Komponenten (B) bzw. in einem Bereich um den Rand der zweiten Komponente(n) (B) herum auf die Oberfläche der Komponente (A) erfolgt.

10. Probenaufnahmevorrichtung (1) nach einem der vorhergehenden Ansprüche, wobei das Anbringen der dritten Spritzgusskomponente (C) durch Einspritzen in eine oder mehrere in der zweiten Komponente (B) ausgebildeten Nuten erfolgt, wobei die Nuten zur Oberfläche der ersten Komponente (A) ausgerichtet ist.

11. Verfahren zur Herstellung einer Probenaufnahmevorrichtung (1), insbesondere einer Probenaufnahmevorrichtung (1) nach Anspruch 9, mit folgenden Schritten: (a) Herstellen der ersten Komponente (A), insbesondere in einem Spritzgussverfahren, (b) Herstellen der zweiten Komponente(n) (B), insbesondere in einem Spritzgussverfahren, (c) Auflegen der zweiten Komponente(n) B zum Abdecken der Öffnung(en) der Aussparungen (21, 22) der ersten Komponente (A), und (d) Einbringen der dritten Komponente (C) in die Zwischenräume zwischen den zweiten Komponenten (B) bzw. in einem Bereich um den Rand der zweiten Komponente(n) (B) herum auf die Oberfläche der Komponente A.

12. Verfahren zur Herstellung einer Probenaufnahmevorrichtung (1), insbesondere einer Probenaufnahmevorrichtung (1) nach Anspruch 10, mit folgenden Schritten: (a) Herstellen der ersten Komponente A, insbesondere in einem Spritzgussverfahren, (b) Herstellen der zweiten Komponente(n) B, insbesondere in einem Spritzgussverfahren, (c) Auflegen der Komponente(n) B zum Abdecken der Öffnung(en) der Aussparungen (21, 22) der ersten Komponente (A), wobei wenigstes eine Nut (33, 34) zur Oberfläche des Grundkörpers (2) ausgerichtet ist, und (d) Einbringen der dritten Komponente (C) in die in der zweiten Komponente (B) ausgebildete Nut (33, 34) auf die Oberfläche der ersten Komponente (A), wobei das Einbringen vorzugsweise über wenigstens eine Öffnung (35A-35D) erfolgt, die die Nut (33, 34) mit der exponierten Oberseite der zweiten Komponente (B) verbindet.

13. Verfahren nach Anspruch 11 oder 12, wobei die erste Komponente (A) und die zweite(n) Komponente(n) (B) in den Verfahrensschritten (a) und (b) insbesondere zeitgleich in demselben Werkzeug hergestellt werden.

14. Verfahren nach einem der Ansprüche 11 bis 13, wobei die im Wesentlichen aus den drei Komponenten (A, B, C) bestehende Probenaufnahmevorrichtung (1) in einem Mehrkompopnentenspritzgussverfahren hergestellt wird.

15. Verfahren nach einem der Ansprüche 11 bis 14, wobei die Herstellung der im Wesentlichen aus den drei Komponenten (A, B, C) bestehenden Probenaufnahmevorrichtung (1) nacheinander oder in einem mehrstufigen Spritzgussverfahren erfolgt.

16. Verfahren nach einem der Ansprüche 11 bis 15, wobei die Komponenten (A, B, C), insbesondere die erste und zweite Komponente (A, B), aus demselben Material gefertigt sind, und/oder das Material der dritten Komponente (C) gleich oder unterschiedlich von dem Material der ersten Komponente (A) und/oder dem Material der zweiten Komponente (B) ist.
